# EUROPÄISCHE PATENTSCHRIFT

(11) **EP 3 285 643 B1**
(45) Veröffentlichungstag und Bekanntmachung des Hinweises auf die Patenterteilung: **06.05.2026**
(21) Anmeldenummer: 16711509.6
(22) Anmeldetag: 10.03.2016
(51) Int. Cl.: A61B 5/00, A61B 5/0537, A61B 5/252

(54) **KÖRPERIMPEDANZ-MESSGERÄT**
BODY IMPEDANCE MEASURING DEVICE
APPAREIL DE MESURE DE L'IMPÉDANCE CORPORELLE

(30) Priorität: 22.04.2015 AT 503172015
(43) Veröffentlichungstag der Anmeldung: 28.02.2018
(73) Patentinhaber: Skrabal, Falko, 8043 Graz (AT)
(72) Erfinder: GLITZNER, Philipp, 8054 Seiersberg (AT)
(74) Vertreter: Margotti, Herwig Franz
(86) Internationale Anmeldenummer: PCT/AT2016/050054
(87) Internationale Veröffentlichungsnummer: WO 2016/168873

(56) Entgegenhaltungen:
- EP-A1- 1 731 092
- WO-A1-2014/128237
- US-A1- 2013 102 920
- US-B1- 6 321 112

## Beschreibung

In jüngster Zeit hat sich bei der Entwicklung von medizinischen Körperimpedanz-Messgeräten eine Tendenz gebildet, die Vermessung von Körperkompartimenten mit Hilfe von Ganzkörperimpedanz zugunsten einer Vermessung der Körperkompartimente mit Hilfe von segmentalen Impedanzmessungen hintanzustellen. Bei der Ganzkörperimpedanz betrachtet man den menschlichen oder tierischen Körper als aus verschiedenen, elektrisch leitfähigen Körperteilen zusammengesetzt und versucht mittels mathematischer Modelle die unterschiedlichen Durchmesser und Längen dieser elektrischen Leiter (nämlich Arme, Beine und Rumpf) zu berücksichtigen. Da der Widerstand eines elektrischen Leiters abgesehen von seinem spezifischen Widerstand in erster Linie von dessen Länge und Querschnitt abhängt, muss man bei dieser Methode fixe Verhältnisse von Körpergröße zu den Durchmessern und Querschnitten von Rumpf und Extremitäten annehmen. Dazu werden üblicherweise Standardproportionen des menschlichen Körpers herangezogen, die z.B. aus Vermessungen an Personal der US Armee stammen. Diese Modelle und Annahmen bringen allerdings derartige Ungenauigkeiten in die Methode, dass diese zwar näherungsweise zur Untersuchung von gesunden Personen, nicht jedoch bei kranken Menschen anwendbar ist.

Mit der segmentalen Impedanzmessung von Körperkompartimenten, speziell wenn diese bei mehreren unterschiedlichen Frequenzen durchgeführt wird, kann man die unterschiedlichen Dimensionen der Körperteile und deren Auswirkungen auf die Wechselstrom-Leitfähigkeit besser berücksichtigen. Mit der segmentalen Impedanzmessung wird besonders versucht, mit Hilfe von Mehrfrequenzmessungen zwischen intrazellulärem und extrazellulärem Wasser zu unterscheiden. Trotz aller Fortschritte auf dem Gebiet ist es bis heute aber nicht gelungen, diese Methoden in die Routinebetreuung am Krankenbett einzuführen. Eine Ausnahme stellt die Messung von Lymphödem an einzelnen Extremitäten dar, bzw. der Versuch, Über- und Unterhydrierung von Dialysepatienten an einem Körperteil, nämlich dem Unterschenkel zu quantifizieren.

Für die segmentale Impedanzanalyse wurden zahlreiche Patente angemeldet. Beispielsweise wird bei dem in der WO 2007/002991 (Chetham) und in der US 8781551 (Chetham) vorgeschlagenen Impedanzmessgerät der Mess-Strom gemessen und anschließend nachgeregelt, es sind keinerlei Umschaltmöglichkeiten vorgesehen. In der US 8594781 (Chetham) ist ein Impedanzmessgerät offenbart, bei dem Messelektroden weggeschaltet werden, um induktive Störungen zu beseitigen. Beim Impedanzmessgerät gemäß der US 2011/0087129 (Chetham) bestimmt ein erstes System die Impedanzprozedur, selektiert Anweisungen, ein zweites System generiert entsprechend diesen Anweisungen Kontrollsignale. Chatham in US 8548580 schlägt zwei Systeme vor, von denen das erste die Instruktionen für die Messung selektiert und diese Instruktionen zu einem zweiten System transferiert. In der Patentanmeldung US 2008/0009757 A1 (Tsoglin) ist ein Impedanzmessgerät offenbart, bei dem die Stromdistorsionen in nicht gemessenen Körpersektionen entsprechend einem elektrischen Modell berücksichtigt werden, bei dem es sich offensichtlich um die Kirchhoff Regeln handelt. Cha in US 8386028 untersucht zwei Segmente gleichzeitig bei zwei verschiedenen Frequenzen. In der US 2011/0046505 (Cornish) werden zwei verschiedene Körpersegmente mit einander verglichen.

Die WO 2008/031030 (Bartnik) offenbart die Gewinnung systolischer Zeitintervalle, indem von einer ersten Kurvenform, welche aus einem Impedanzsignal stammt, eine zweite Kurvenform subtrahiert wird, die aus Echokardiographie oder aus der Pulswelle oder dem Pulsoximeter gewonnen wird. Die WO 2006/063255A2 (Bernstein) offenbart, aus dem Impedanzsignal über dem Thorax oder über der Brachialarterie das Schlagvolumen zu bestimmen. Die US 2013/0096448 (Brooks) beschreibt eine kombinierte ECG-(Elektrokardiographie-), ICG- (Impedanzkardiographie-) und Phonoelektrode auf einem gemeinsamen Träger mit akustischer Kammer. Die Schriften US 8521264 und US 2010/0324404 beschreiben die Verwendung von maximal drei kombinierten ECG-ICG-Elektroden, die alle am Thorax platziert sind. Die US 6339722 (Heethaar) schlägt vor, den Thorax als ein Segment bei zwei Frequenzen und mit zwei verschiedenen Messdistanzen zu vermessen, um Information über die Herztätigkeit zu gewinnen.

Die Schriften WO2014/128237 A1, EP 1 731 092 A1 und US 6,321,112 B1 offenbaren weitere Vorrichtungen zur Impedanzmessung an Segmenten eines menschlichen oder tierischen Körpers.

Für eine rasche und wenig umständliche Durchführung einer multisegmentalen Mehrfrequenzimpedanzanalyse ist es notwendig, automatisch und zweckmäßig ohne Zutun des Anwenders zahlreiche Körpersegmente zu vermessen. Dafür ist üblicherweise ein Multiplexer vorgesehen, der in das Gerät eingebaut ist. Multiplexer für die Impedanzmessung werden teilweise bei den oben zitierten Impedanzmessgeräten eingesetzt, um an verschiedenen Stellen des Körpers Strom einzuspeisen und andererseits an verschieden Stellen des Körpers die Spannung zu messen.

Dabei ergeben sich jedoch einige, in den oben zitierten Schriften nicht beachtete Schwierigkeiten. Für viele Anwendungen muss der Abstand zwischen dem Messgerät und dem untersuchten Körper groß gehalten werden, wodurch sich auch relativ große Kabellängen ergeben. Dies kann wesentliche Schwierigkeiten und Fehler bei der Messung verursachen. So können durch die Kabel und ihre Abschirmungen parasitäre Kapazitäten und Induktivitäten entstehen. Untersuchungen des Anmelders haben gezeigt, dass insbesondere verhindert werden muss, dass durch gleichzeitig an den Körper angelegte Kabel parasitäre Leckströme in die zusätzlich angelegten Kabel fließen, die zum Zeitpunkt der Anwendung nicht für die Stromeinspeisung vorgesehen sind. Weiters muss verhindert werden, dass durch die kombinierten Einspeise- und Messkabel, die zu zahlreichen Einspeise- und Messpunkten führen, Streuimpedanzen und Streukapazitäten eine Verfälschung der gemessenen Signale in den einzelnen Kabeln verursachen.

Es ist somit die Aufgabe der vorliegenden Erfindung eine Vorrichtung zu schaffen, die diese Schwierigkeiten und Nachteile des Standes der Technik umgeht.

Die vorliegende Erfindung löst diese Aufgabe durch eine Vorrichtung mit den Merkmalen des Anspruchs 1. Vorteilhafte Ausführungsformen der Erfindung sind in den Unteransprüchen dargelegt.

Die erfindungsgemäße Vorrichtung zur Impedanzmessung an Segmenten eines menschlichen oder tierischen Körpers, wobei die Impedanzmessung bevorzugt eine Multifrequenz-Impedanzmessung ist, umfasst eine Stromquelle, Stromumschalter, an Segmenten des menschlichen oder tierischen Körpers anordenbare Stromelektroden und Messelektroden. Ein jeder Ausgang der Stromquelle ist mit einem Eingang eines Stromumschalters verbunden, wobei jeder Stromumschalter eine Vielzahl von Ausgängen aufweist und der Eingang des jeweiligen Stromumschalters umschaltbar mit einem seiner Ausgänge zusammenschaltbar ist, wobei die Ausgänge der Stromumschalter mit elektrischen Speiseleitungen der Stromelektroden verbunden sind. Die Messelektroden erfassen Spannungssignale und die Vorrichtung ermittelt aus dem von der Stromquelle gelieferten Strom und den erfassten Spannungssignalen die Impedanzen der Segmente des menschlichen oder tierischen Körpers. In die Speiseleitungen sind nahe der oder unmittelbar an den Stromelektroden Schaltvorrichtungen eingebaut, mit denen die Stromelektroden mit ihren Speiseleitungen verbindbar und von ihnen trennbar sind. Die Vorrichtung ist dazu konfiguriert, durch Schalten der Stromumschalter und der Schaltvorrichtungen jeweils nur zwei Stromelektroden mit der Stromquelle zu verbinden und alle übrigen Stromelektroden durch Schalten der Schaltvorrichtung von der Speiseleitung zu trennen und die Speiseleitung durch Schalten der Stromumschalter von der Stromquelle zu trennen.

Bevorzugt sind nahe oder unmittelbar an den Messelektroden Spannungsverstärker bzw. Spannungsfolger mit hohem Eingangswiderstand in die Signalleitungen eingefügt.

Für automatische Messvorgänge sollten die Schaltvorrichtungen von der Vorrichtung schaltbar ausgeführt sein.

Für die automatische Messung von Impedanzen an unterschiedlichen Körpersegmenten ist in einer Ausführungsform der Erfindung vorgesehen, dass Signalleitungen der Messelektroden zu Eingängen von Spannungsumschaltern geführt sind, wobei die Spannungsumschalter jeweils einen Ausgang aufweisen, der mit einem Eingang des Spannungsumschalters zusammenschaltbar ist. Mit dieser Konfiguration kann Impedanzmessung vereinfacht werden, indem die Ausgänge der Spannungsumschalter mit Eingängen eines Differenzverstärkers verbunden sind, der aus den an seinen Eingängen anliegenden Spannungssignalen eine Differenzspannung ermittelt, die die Vorrichtung für die Impedanzermittlung verwendet.

Hohe Verlässlichkeit und schnelle Messungen erzielt man, indem die Stromumschalter und/oder die Spannungsumschalter als Relais oder Multiplexer ausgebildet sind.

Für eine wesentliche Verbesserung der Messergebnisse ist gesorgt, wenn die erfindungsgemäße Vorrichtung mit einer Gleichtaktunterdrückung ausgestattet wird. Dazu ist erfindungsgemäß vorgesehen, dass zumindest eine der Stromelektroden über einen Widerstand mit einem Ausgleichsstrom beaufschlagbar ist, wobei der Anschluss des Widerstands an die Stromelektrode zwischen der Schaltvorrichtung und der Stromelektrode angeordnet ist. Alternativ oder ergänzend dazu ist eine Ausgleichsstromelektrode vorgesehen, die über einen Widerstand mit einem Ausgleichsstrom beaufschlagbar ist.

Die Handhabung der erfindungsgemäßen Vorrichtung wird für das Untersuchungspersonal besonders einfach, wenn, abgesehen von eventuell vorhandenen Einzelmesselektroden, jeweils eine Stromelektrode und eine Messelektrode zu einer vereinten Elektrodenregion gruppiert sind. Die Handhabung der Vorrichtung wird weiter vereinfacht, indem die Stromelektrode und die Messelektrode der vereinten Elektrodenregion gemeinsam geführte Kabel als Speiseleitung und Signalleitung aufweisen, da durch diese Maßnahme nur mit der halben Zahl an Kabeln hantiert werden muss. Eine weitere Vereinfachung der Handhabung ergibt sich, indem die Stromelektrode und die Messelektrode auf einem gemeinsamen Elektrodenträger angeordnet sind. Der gemeinsame Elektrodenträger ist bevorzugt eine Klemme, ein Band, eine Manschette oder auch eine Druckmanschette (ähnlich den für die Blutdruckmessung verwendeten Druckmanschetten). Wenn eine Klemme, Manschette oder Druckmanschette verwendet wird, kann an dieser auch ein Drucksensor zur Pulsmessung angeordnet werden. Dabei ist es für die Durchführung automatischer Messungen vorteilhaft, wenn der Drucksensor eine flüssigkeitsgefüllte Blase aufweist, die mit einem Druckaufnehmer kommuniziert, wobei vorzugsweise die flüssigkeitsgefüllte Blase mittels einer gesteuerten, hydraulischen oder elektromotorischen Anpressvorrichtung gegen ein Körperteil anpressbar ist.

Es hat sich gezeigt, dass die Genauigkeit der Messungen wesentlich erhöht wird, wenn die Vorrichtung nur von maximal einer vereinten Elektrodenregion die Stromelektrode mit der Stromquelle zusammenschaltet und die Messelektrode zur Spannungsmessung verwendet. In dieser Hinsicht als vorteilhaft hat sich erwiesen, wenn vereinte Elektrodenregionen zur Anbringung an peripheren Körpersegmenten und an zentralen Körpersegmenten auswählbar sind, wobei eventuell auch eine weitere vereinte Elektrodenregion zum Anbringen an einer Körperregion auswählbar ist, an der die Brustwand-Elektroden V1 bis V6, bevorzugt V4 bis V6, eines EKGs platziert sind. In der einfachsten Anwendung muss jedoch nur eine der Brustwandelektroden V1-V6, bevorzugt V4 bis V6, als Messelektrode für die Impedanzmessung verwendet werden.

Weiters ist es zweckmäßig, wenn die Vorrichtung mit einem Differentiator zur Bestimmung der Änderung der Impedanz mit dem Herzschlag ausgestattet ist.

In einer bevorzugten Ausführungsform der erfindungsgemäßen Vorrichtung ist ein EKG-Gerät in die Vorrichtung integriert, wobei das EKG-Gerät zumindest Extremitätenelektroden, vorzugsweise auch Brustwandelektroden, aufweist. Damit ist es möglich, mit der erfindungsgemäßen Vorrichtung gleichzeitig Impedanzmessungen und EKG-Aufzeichnungen durchzuführen. Zur Minimierung der Zahl der am Körper anzubringenden Elektroden schlägt die Erfindung auch vor, dass die Messelektroden als EKG-Elektroden ausgebildet sind, indem Zweigleitungen der Signalleitungen der Messelektroden zum EKG-Gerät geführt sind.

Wenn die Stromelektroden und/oder die Messelektroden als Aufnehmer und/oder Geber für physikalische Größen, insbesondere Beschleunigungswerte, Druck, Schall, Temperatur oder Licht, ausgebildet sind, können während der Impedanzmessungen auch andere physikalische Größen und Parameter gemessen werden. Verlässlichkeit der Messung und einfache Handhabung wird erreicht, indem zumindest einige der Stromelektroden und der Messelektroden als Saugelektroden oder Klebeelektroden ausgebildet sind.

Die Erfindung wird nachfolgend anhand von Ausführungsbeispielen unter Bezugnahme auf die Zeichnungen näher erläutert.
Fig. 1 zeigt ein schematisches Blockschaltbild der erfindungsgemäßen Vorrichtung.
Fig. 2 zeigt Anwendungsbeispiele der erfindungsgemäßen Vorrichtung.

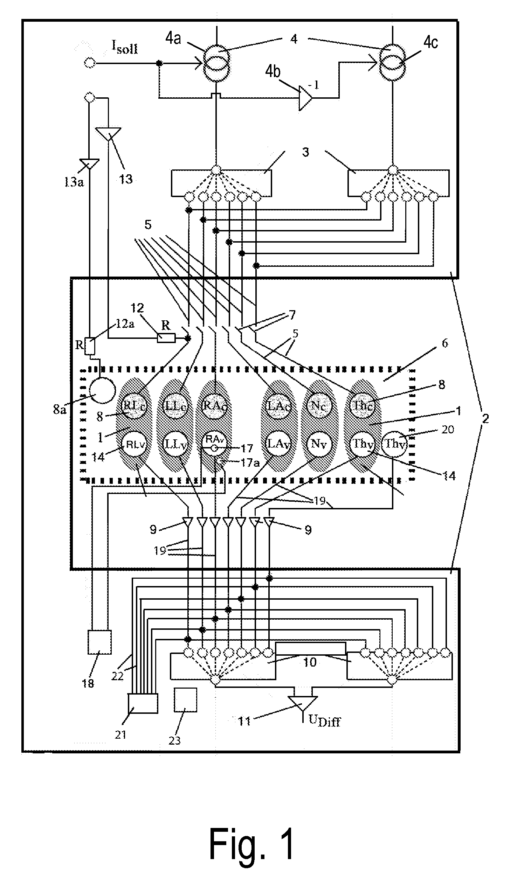

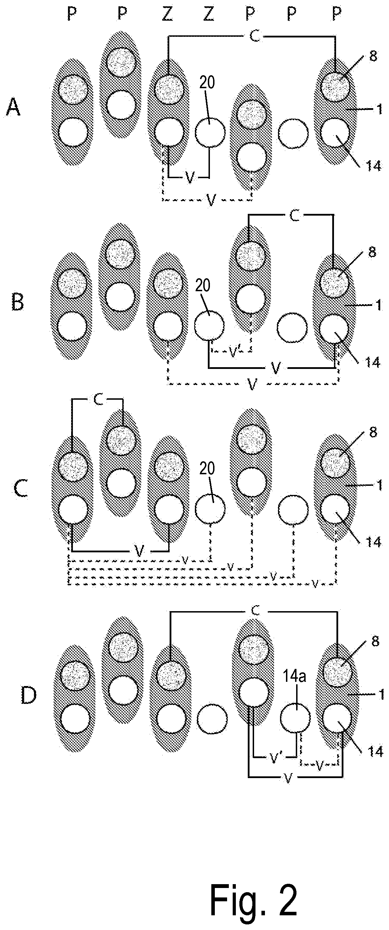

In der nachfolgenden Beschreibung wird auf Fig. 1 und Fig. 2 Bezug genommen. In jeder der beiden Figuren sind der Übersichtlichkeit halber mehrfach nebeneinander dargestellte gleiche Teile, die eine identische Konfiguration aufweisen, jeweils nur einmal mit einem Bezugszeichen versehen.

Fig. 1 zeigt ein schematisches Blockschaltbild der erfindungsgemäßen Vorrichtung 2 zur Messung von Impedanzen von Segmenten eines menschlichen oder tierischen Körpers. Die Vorrichtung 2 umfasst eine Stromquelle 4 mit zwei durch einen Steuerstrom Isoll regelbaren Teilstromquellen 4a, 4c von gegensätzlicher Polarität, indem der Steuerstrom Isoll zu einer der Teilstromquellen 4c einen Inverter 4b durchläuft, bevor er in diese Teilstromquelle 4c eintritt. Der Ausgang einer jeden Teilstromquelle 4a, 4c ist mit dem Eingang eines Stromumschalters 3 verbunden, der z.B. als Multiplexer ausgeführt ist. Jeder Stromumschalter 3 weist eine Vielzahl von Ausgängen auf, wobei der Eingang umschaltbar mit einem der Ausgänge zusammengeschaltet ist. Die Ausgänge der Stromumschalter 3 sind mittels elektrischer Speiseleitungen 5 mit Stromelektroden 8 verbunden. Die Stromelektroden 8 sind mit punktierter Fläche gezeichnet. Die Stromelektroden 8 werden an einzelnen Körpersegmenten des untersuchten Lebewesen (Mensch oder Tier) aufgebracht, Die Oberfläche des untersuchten Lebewesens 6 ist in Fig. in strichlierter Umrandung symbolisch dargestellt. Nahe den Stromelektroden 8 sind in die Speiseleitungen 5 Schaltvorrichtungen 7 eingebaut, mit denen die Stromelektroden 8 von der Speiseleitung 5 zu- und weggeschaltet werden können. Dies dient dazu, im Betrieb der Vorrichtung 2 parasitäre Leckströme nicht von den Stromelektroden 8 zur Vorrichtung 2 gelangen zu lassen, indem die Schaltvorrichtungen 7 im weggeschalteten Zustand für eine Trennung der Stromelektroden 8 von den Speiseleitungen 5 sorgen. Die Schaltvorrichtungen 7 können als mechanische Schalter (z.B. Mikrorelais), elektronische Schalter oder auch als negative Impedanzkonverter ausgebildet sein. Somit wird jede Speiseleitung 5 einerseits in der Vorrichtung 2 durch den Stromumschalter 3 von der Stromquelle 4 weggeschaltet und andererseits zusätzlich durch die Schaltvorrichtung 7 peripher von der Vorrichtung 2 ein zweites Mal von der Stromelektrode 8 weggeschaltet, wodurch parasitäre Leckströme, die über den Körper des Lebewesens von der Peripherie her in die Kabel der Speiseleitungen 5 gelangen könnten, unterbunden werden. Dies ist besonders wichtig, weil die Kabel der Speiseleitungen 5 vorteilhaft geschirmt und - für eine noch bessere Schirmwirkung - mit einer aktiven Schirmung versehen werden sollten. Dadurch könnte allerdings auch über Kapazitäten zwischen Kabel und Schirm generierter Wechselstrom fließen, was durch die Schaltvorrichtungen 7 verhindert wird.

Im Ausführungsbeispiel von Fig. 1 sind sechs Stromelektroden 8 vorgesehen. Die ersten vier Stromelektroden 8 sind beispielhaft für die Stromeinspeisung am rechten Bein (Bezeichnung RL_{C} für right leg current), am linken Bein (Bezeichnung LL_{C} für left leg current), am rechten arm (Bezeichnung RA_{C} für right arm currrent) und am linken Arm (Bezeichnung LA_{C} für left arm current) vorgesehen. Die fünfte, optional anzubringende Stromelektrode 8 ist für die Anbringung an der oberen Thoraxapertur, z.B. nahe des oberen Sternums, am Hals oder am Kopf zur Einspeisung des Stroms vorgesehen (Bezeichnung N_{C} für Neck current). Die sechste Stromelektrode 8 ist für die Stromeinspeisung am unteren Ende des Thorax (Bezeichnung Th_{C}) vorgesehen, z.B. in der Region, wo für ein EKG die Ableitungen V4 bis V6 oder V4r bis V6r angeordnet werden.

Jeder Stromelektrode 8 ist eine Messelektrode 14 zugeordnet, wobei die Stromelektrode 8 und die Messelektrode 14 elektrisch nicht miteinander verbunden sind und die Kombination aus Stromelektrode 8 und Messelektrode 14 am Lebewesen 6 in einer sogenannten vereinten Elektrodenregion 1 angeordnet ist. Der Abstand der Elektroden 8, 14 der vereinten Elektrodenregion sollte minimal 2 cm bis 4 cm, besser 3 cm bis 4 cm nicht unterschreiten und maximal 20 bis 30 cm nicht überschreiten. Im Ausführungsbeispiel von Fig. 1 gibt es als Beispiel somit sechs vereinte Elektrodenregionen 1 mit jeweils einer Stromelektrode 8 und einer Messelektrode 14 sowie zumindest eine einzelne zusätzliche Messelektrode 20.

Die Messelektroden 14 sind mit RL_{V} für right leg voltage, LL_{V} für left leg voltage, RA_{V} für right arm voltage, LA_{V} für left arm voltage, N_{C} für neck voltage und Th_{V} für thorax voltage bezeichnet. Jede Messelektrode 14 kann zusammen mit der zugeordneten Stromelektrode 8 an einem geeigneten gemeinsamen Elektrodenträger, wie z.B. einer Klemmelektrode, Klebeelektrode, Saugelektrode, Bandelektrode, Manschettenelektrode oder Druckmanschettenelektrode, angeordnet sein. Alternativ dazu liegen die Messelektroden 14 und Stromelektroden 8 als separate Einzelelektroden vor.

Die Messelektroden 14 sind mit Signalleitungen 19 verbunden, die zu Eingängen von zwei Spannungsumschaltern 10 geführt sind, wobei in die Signalleitungen 19 Spannungsverstärker 9 geschaltet sind. Die Spannungsverstärker 9 können als Spannungsfolger mit hohem Eingangswiderstand ausgeführt sein. Die Spannungsverstärker 9 sollten möglichst nahe oder unmittelbar an den Messelektroden 14 angeordnet sein. Die Spannungsumschalter 10 sind beispielsweise als Relais oder elektronische Spannungsmultiplexer ausgeführt. Mittels der Spannungsumschalter 10 kann jeweils ein Signal einer Messelektrode 14 auf die Ausgänge der Spannungsumschalter 10 geschaltet werden, wo es dann für die weitere Verarbeitung z.B. zu einem Eingang eines Differenzverstärkers 11 geleitet wird. Das Ausgangssignal UDiff des Differenzverstärkers ist die Differenz der Ausgangssignale der beiden Spannungsumschalter 10.

Eine weitere wichtige Voraussetzung für die optimale Funktion der Vorrichtung 2 ist das Vorsehen einer Gleichtaktunterdrückung ("common mode rejection"), da eine große elektromagnetische Interferenz durch das untersuchte Lebewesen 6 besteht. Auch Wechselstrominterferenz, verursacht durch das Spannungsversorgungsnetz, kann große Störungen verursachen. Weiters muss eine Fehlanpassung der verschiedenen Kabelkapazitäten und Kabelimpedanzen der Speiseleitungen 5 und Signalleitungen 19 ausgeglichen werden. Neben den üblichen Methoden einer optimalen Abschirmung der Kabel, auch unter Verwendung einer aktiven Schirmung, Verwendung besonderer Signalfiltertechniken, wie adaptiven Filtern oder "Notch"-Filtern, bedarf es eines Ausgleichs der Gleichtaktspannung ("common mode voltage"), indem ein Ausgleichsstrom durch einen hohen Widerstand 12 mit einem Widerstandswert zwischen 470 kOhm und 1,5 MOhm, vorzugsweise 1 MOhm, getrieben wird. Dazu muss allerdings ein Anschluss des Widerstands 12 mit zumindest einer der Speiseleitungen 5 zu den Stromelektroden 8 verbunden sein, und zwar zwischen der Schaltvorrichtung 7 und der Stromelektrode 8. Der andere Anschluss des Widerstands 12 ist mit dem Ausgang eines Operationsverstärker 13 verbunden, der den Ausgleichsstrom liefert. Der Widerstand 12 wird vorteilhaft knapp neben der für die Einspeisung des Ausgleichsstroms verwendeten Stromelektrode 8 platziert. Jede der Stromelektroden 8 ist für die Einspeisung des Ausgleichsstroms geeignet. Alternativ oder zusätzlich kann eine dezidierte Ausgleichsstromelektrode 8a vorgesehen werden, die über einen Widerstand 12a (zwischen 470 kOhm und 1,5 MOhm, vorzugsweise 1 MOhm) mit dem Ausgang eines Operationsverstärkers 13a verbunden ist, der in bekannter Weise den Ausgleichsstrom bereitstellt.

Mit der solcherart aufgebauten Vorrichtung 2 können automatisch viele Segmente des Lebewesens 6 genau untersucht werden. Einerseits ist es möglich, mithilfe des Steuersignals Isoll und der beiden invertiert betriebenen Teilstromquellen 4a, 4c Wechselströme beliebiger Frequenz zu generieren und diese mittels der beiden Stromumschalter 3 an frei selektierbare Stromelektroden 8 zu leiten. Andererseits können mithilfe der beiden Spannungsumschalter 10 Spannungssignale von frei selektierbaren Messelektroden 14 zur weiteren Verarbeitung, insbesondere zur Differenzbildung am Differenzverstärker 11, ausgewählt werden. Die Steuerung des Steuersignals Isoll, der Stromumschalter 3, der Spannungsumschalter 10, die mathematische Verarbeitung der von den Messelektroden 14 ermittelten Spannungssignale sowie die Steuerung aller weiterer Funktionen der Vorrichtung wird mittels einer in der Vorrichtung 2 eingebauten CPU 23 durchgeführt, die beispielsweise in Form eines Microcontrollers ausgeführt ist. Die zahlreichen notwendigen Zuleitungen zur CPU 23 sind im Sinne der Übersicht nicht gezeichnet.

Somit ist es möglich, mittels der Vorrichtung 2 automatisch Impedanzmessungen bei unterschiedlichen Frequenzen an unterschiedlichen Segmenten des Lebewesens 6 durchzuführen. Auf Basis dieser Impedanzmessungen an unterschiedlichen Körpersegmenten bietet die Vorrichtung 2 auch die Möglichkeit von Impedanzkardiographie (IKG).

Weiters kann die CPU 23 in der Vorrichtung 2 auf Basis der Impedanzmessungen mit eingespeicherten, empirischen Gleichungen oder mit vorgegebenen mathematischen Modellen die Körperzusammensetzung des Ganzkörpers des Lebewesens 6 und seiner Körperteile, wie z.B. das in Körperteilen enthaltene Körperwasser, die extrazelluläre Flüssigkeit, die Muskelmasse, die Fettmasse, deren Abweichungen vom Sollwert, sowie Ödeme oder Flüssigkeitsansammlungen in Körperteilen errechnen und die berechneten Ergebnisse zur Anzeige an einem Bildschirm, zur Speicherung in einem nichtflüchtigen Speicher oder zur weiteren Verarbeitung in Datenbanken ausgeben. Es ist zu betonen, dass der Arzt mithilfe der Vorrichtung 2 eine Diagnose einer Abweichungen der Hydrierung von der Norm, z.B. Abweichungen in der Relation zwischen Fettmasse oder "Lean Body Mass" zum Extrazellulärvolumen oder zum Verhältnis extrazelluläres zu intrazellulärem Voluem bzw. Ganzkörperwasser, vornehmen und somit besser Überhydrierung oder Dehydrierung feststellen kann. Weiters können auch arterielle Durchblutungsstörungen, vor allem der Beine oder deren Segmente, erkannt und ausgegeben werden. So kann aus dem Unterschied der Beschleunigung des Volumens der Beine mit dem Herzschlag auch ein "Ankle Brachial Index" (ABI), wie er aus der Literatur bekannt ist, errechnet werden. Die durch die Vorrichtung 2 ermittelten Impedanzen, sowie deren Wirkwiderstand- und Blindwiderstandanteil und der Phasenwinkel der Impedanz dienen dazu, mittels multipler Regressionen oder auch neuronalen Netzwerken Regressionsgleichungen zu entwickeln, die den Arzt interessierenden Parameter zu schätzen, und die mittels Goldstandardverfahren wie Ganzkörper DXA, Deuteriumdilution, Natriumbromid Dilution oder anderen Tracern ermittelten Parameter vorherzusagen.

Wenn die Vorrichtung 2 die ermittelten Impedanzsignal zusätzlich differenziert und mit dem Herzschlag in Beziehung gesetzt wird, kann die solcherart berechnete Änderung der Impedanz mit dem Herzschlag verwendet werden, um die Beschleunigung des Blutes in verschiedenen Körperteilen zu messen. Dies bewährt sich besonders dann, wenn es in einem Segment, definiert durch eine zentrale vereinte Elektrodenregion einerseits (z.B. am Hals, Nacken oder Schultern angeordnet) und einer weiteren zentralen Einzelelektrode (gewählt zB aus V4 bis V6 oder aus V4r bis V6r) und einer peripheren vereinten Elektrodenregion und einer zentralen und einer peripheren vereinten Elektrodenregion andererseits erfolgt, wie in Skrabal et al, Medical Engineering & Physics 36 (2014) 896-904 beschrieben. Wesentlich ist nur, dass zumindest eine Messelektrode 14 zwischen zwei Stromelektroden 8 zu liegen kommt. So kann nicht nur die Herzleistung beurteilt werden und eine Herzschwäche diagnostiziert werden, sondern auch im Sinne der Impedanzrheographie die Messung der arteriellen und venösen Durchblutung bzw. auch der Pulswellenlaufzeit (Volumswellenlaufzeit) erfolgen. Auch hier werden vorteilhaft Goldstandardverfahren wie Echocardiographie und andere hämodynamische Methoden wie Ankle Brachial Index, Pulswellenlaufzeit, Pulswellenanalyse mit Augmentationsindex, zentraler und peripherer Compliance, Schlagvolumen aus Pulskurve als Goldstandardverfahren eingesetzt, um die neuen Methoden zu kalibrieren.

In die Vorrichtung 2 kann auch ein EKG-Gerät 21 integriert sein. Dieses EKG-Gerät 21 kann mit eigenen EKG-Elektroden ausgestattet sein, die aus Gründen der Übersichtlichkeit in Fig. 1 nicht dargestellt sind. Als besonderen Vorteil bietet die Vorrichtung 2 jedoch auch die Möglichkeit, die Messelektroden 14 als EKG-Elektroden auszuführen, indem, vorzugsweise zwischen Spannungsverstärker 9 und Spannungsumschalter 10, Zweigleitungen 22 von den Signalleitungen 19 der Messelektroden 14 zum EKG-Gerät 21 geführt werden. Damit kann die Vorrichtung 2 als Mehrkanal EKG-Gerät dienen und während der Aufzeichnung eines Mehrkanal-EKGs gleichzeitig die beschriebenen Impedanzmessungen und Berechnungen aus den ermittelten Impedanzen durchführen. Dadurch, dass für die Gewinnung der Templates eine Aufzeichnung über einige Zeit, z.B. 2 bis 3 Minuten günstig ist, wird automatisch auch ein Rhythmusstreifen generiert, wie er auch für das übliche EKG gewünscht wird und üblich ist. Dadurch wird auch eine Spektralanalyse der Herzintervalle und der Änderungen der Impedanz mit dem Herzschlag möglich. So können dann auch über die entsprechenden Frequenzbänder, zB mittels des 0,1 Hz Bands und des 0,3 Hz Bands die Anteile von Sympathicus und Vagus für Herzfrequenzsteuerung ermittelt werden, was sich zB besonders zur Feststellung von Übertraining, Burnout, Depression usw eignet, vor allem wenn es im Zeitverlauf über Wochen und Monate verfolgbar ist. Dies alles ist mit keinem erhöhten Zeitaufwand gegenüber dem üblichen Rhythmusstreifen für das EKG verbunden.

Die Erfindung sieht auch vor, dass an den Stromelektroden 8 oder Messelektroden 14 Sensoren bzw. auch Aktoren angebracht sind, wie z.B. Accelerometer, Drucksensoren, oder Lichtsensoren, LEDS oder Druckpumpen. In Fig. 1 ist ein Messgrößenaufnehmer/Geber 17 schematisch direkt unter einer Elektrode (Strom- oder Messelektrode können verwendet werden) liegend gezeichnet, welcher optional auch als neben der Elektrode liegender Messgrößenaufnehmer und/oder Geber 17a ausgeführt sein kann. Der Messgrößen-Sensor 7 und/oder Geber 17, 17a wird von einer Auswerteeinheit 18 angesteuert, die in diesem Fall auch als Generator für die einzuprägenden Messgröße wirkt, z.b. ein Drucksignal erzeugt. Der Aufnehmer/Geber 17, 17a ist mit einem Eingang der Auswerteeinheit 18 verbunden, die die aufgenommene Messgröße, z.B. ein Drucksignal, Beschleunigungssignal, Temperatursignal, etc. auswertet. Mit solchen geschilderten Sensoren kann die Vorrichtung 2 gleichzeitig zur Impedanzmessung und EKG-Aufzeichnung noch andere Kreislaufparameter, wie die Kreislaufzeiten, Pulswellenanalyse inklusive Herzschlagvolumen, Pulsoximetrie usw. erfassen, wobei entsprechende Methoden in der Schrift WO 2004/030535 A1 (Skrabal) und EP 2319411 A2 detailliert offen gelegt sind. Die Pulswellenanalyse kann dann auch zur Berechnung von anderen Parametern, wie Gefäßsteifigkeit, Augmentationsindex, zentraler Aortendruck, Schlavolumen usw verwendet werden. Auch das eventuelle Anbringen von Blutdruckmanschetten, auch an der unteren Extremität zur Bestimmung des Ankle-Brachial Index und zur Venenverschlussplethysmographie, ist vorgesehen.

Somit stellt die Vorrichtung 2 ein Messgerät dar, das in der vollen Ausbaustufe nicht nur die Impedanzmessung an mehreren Körpersegmenten eines menschlichen oder tierischen Körpers bei mehreren oder zahlreichen Impedanzfrequenzen durchführt, sondern gegebenenfalls auch einen Differentiator (der in die CPU 23 integriert sein kann) zur Erfassung der Impedanzänderung mit dem Herzschlag umfasst. Der Herzschlag kann genau aus dem EKG erkannt werden, sodass danach ein Zeitfenster zur Suche nach der Impedanzänderung mit dem Herzschlag festgelegt werden kann. Templates werden aus den verschiedenen Segmenten für Impedanzkardiographie und Impedanzrheographie generiert. Das Messgerät ist also gegebenenfalls auch ein (Vielkanal) EKG Gerät, sowie dient als Analysator für weitere physikalische Größen, sofern die entsprechenden Sensoren und Aktoren in die Elektroden eingebaut sind.

Fig. 2 zeigt anhand von vier Beispielen A, B, C, D die Anwendung des Prinzips der teilweisen oder kompletten Trennung der vereinten Elektrodenregionen 1 für die Stromeinspeisung (C für current) durch die Stromelektroden 8 und Spannungsmessung (V für voltage) durch die Messelektroden 14 der Vorrichtung 2. Wie ersichtlich werden in diesen Beispielen die vereinten Elektrodenregionen 1 für die Stromeinspeisung einerseits und die Spannungsmessung andererseits teilweise oder auch ganz unterschiedlich verwendet. Die Stromelektroden sind hier mit punktierter Fläche, die Messelektroden weiß gezeichnet. Wenn ein Elektrodenpaar anatomisch außerhalb des Stromkreises liegt, ist es auch in der Zeichnung verschoben und außerhalb des Stromkreises gezeichnet.

In einer Ausführungsform der Erfindung bestimmt die Vorrichtung die Impedanz und eine Änderung der Impedanz mit dem Herzschlag in zumindest zwei Körpersegmenten, wobei die Segmente wie folgt definiert sind.
a) Einerseits ist das zentrale Segment bezüglich der Stromeinspeisung definiert durch die Stromelektroden 8 einer zentralen (Z) vereinten Elektrodenregion 1 und einer peripheren (P) vereinten Elektrodenregion 1, die Messtrecke andererseits ist definiert durch die Messelektrode 14 der zentralen vereinten Elektrodenregion 1 und durch eine Messelektrode 20, die auch zur Ableitung des Brustwand EKG verwendet wird. (Fig. 2-A, durchgezogene Messtrecke V.) Hier könnte weniger komfortabel auch eine weitere zentrale vereinte Elektrodenregion 1 verwendet werden, deren Messelektrode 14 zur Messung der Impedanz und deren Stromelektrode 8 zur Einspeisung des Stroms verwendet wird (nicht gezeichnet). Alternativ könnte bei gleicher Einspeisung wie oben die Messtrecke V definiert sein durch die Messelektrode 14 der zentralen vereinten Elektrodenregion und durch die Messelektrode 14 einer vereinten Elektrodenregion, welche nicht inerhalb des Stromkreises liegt (z.B. am kontralateralen Bein positioniert ist). (Siehe strichlierte Messtrecke in Fig. 2 A.)
b) Andererseits ist das periphere Körpersegment in der Stromeinspeisung definiert durch die Stromelektroden 8 von zwei peripheren vereinten Elektrodenregionen (z.B. an beiden Beinen angebracht), die Messtrecke hingegen ist definiert durch eine Messelektrode, die außerhalb des Stromkreises liegt (z.B. die über der Brustwand liegende EKG Elektrode 20) und die Messelektrode 14 der vereinten Elektrodenregion 1, welche auch zur Stromeinspeisung verwendet wurde (Fig. 2-B, durchgezogener Messkreis). Alternativ könnte bei gleicher Einspeisung der Messkreis durch eine außerhalb dieses Stromkreises liegende Messelektrode 14 einer zentralen vereinten Elektrodenregion und durch die Messelektrode 14 der zur Stromeinspeisung benutzten Elektrodenregion definiert sein (Fig. 2-B, strichlierte Messtrecke). Analog zu diesen Ausführungen kann auch ein weiteres Segment zwischen der zentralen Einzelelektrode 20, die einer EKG-Brustwandelektrode entsprechen könnte und der Messelektrode 14 einer vereinten Elektrodenregion, die nicht vom Strom durchflossen ist, analysiert werden (strichlierte Linie mit V'). Dabei könnte es sich z.B. um das Abdomensegment handeln.
c) Fig. 2-C zeigt eine ähnliche Messanordung wie Fig. 2-B, mit der ein weiteres peripheres Körpersegment vermessen werden kann. Hier könnte es sich zB um die Arme handeln. Diese Abbildung veranschaulicht, wieviele verschiedene Messpunkte verwendet werden können, solange diese nur außerhalb des Stromkreises liegen. Dies trifft natürlich auch für die anderen Abbildungen 2-A, 2-B, 2-C zu, wobei dies dort aus Gründen der Übersichtlichkeit nicht eingezeichnet ist. Es sollte die Stromeinspeisung jedoch so erfolgen, dass nicht zwei Körpersegmente mit pulsatiler Änderung des Volumens gemeinsam elektrisch durchströmt und gemeinsam gemessen werden, weil sich die pulsatilen Komponenten vermengen würden. Es wäre dann möglich, aber mühsam, die pulsatilen Komponenten von zwei Abschnitten herauszurechnen.
d) Fig. 2-D zeigt eine Messanordnung, mit der auch Teilsegmente eines peripheren Körpersegmentes vermessen werden können, wenn zwischen peripheren und zentralen Elektrodenpaaren noch zusätzliche einzelne Messektroden 14a angebracht sind. Das dargestellte Messegment V dient zur Messung ders gesamten Segmentes, das dargestellte Messsegment V' dient dabei zur Vermessung des proximalen Teiles und das Segment mit der schraffierten Linie der Vermessung des peripheren Körpersegmentes.

Bei allen Darstellungen in Fig 2 wird im Sinne der Erfindung alternativ immer nur eine Messstrecke durch den Spannungsumschalter 10 freigegeben, auch die Einspeisung erfolgt vorteilhafter Weise immer nur in dem Segment, das gerade untersucht wird, alle anderen Segmente werden durch die zusätzlichen Schaltvorrichtungen 7 und den Stromumschalter 3 weggeschaltet.

So kann mit einem Minumum von Stromumschaltungen jedes beliebige Körpersegment sowohl bezüglich der Impedanz als auch bezüglich der Änderung der Impedanz mit dem Herzschlag vermessen werden. Es ist auch für jeden Fachmann offensichtlich, dass nur ein Bruchtteil der möglichen Alternativen, die dieses Umschaltsystem bietet, dargestellt sind, wobei die Anwendung nicht auf die in den Zeichnungen dargestellten Strom- und Spannungskreise eingeschränkt sind.

Das Wesentliche an den Impedanzmessungen der Vorrichtung 2 ist, dass unter allen vereinten Elektrodenregionen 1 nur von maximal einer vereinten Elektrodenregion sowohl die Stromelektrode 8 zur Stromeinspeisung als auch die Messelektrode 14 zur Spannungsmessung verwendet wird. Bei allen anderen vereinten Elektrodenregionen wird entweder nur eine der beiden Elektroden 8, 14 oder überhaupt keine der beiden Elektroden 8, 14 verwendet.

Was die Realisierung der Elektroden 8, 14 betrifft, so ist es für die Handhabung vorteilhaft, wenn zumindest einige von ihnen als Saugelektroden, Klemmelektroden Klebeelektroden, Bandelektroden, Manschettenelektroden oder Druckmanschettenelektroden ausgeführt sind. Diese können als Spotelektroden, Bandelektroden oder auch Doppelbandelektroden konfiguriert sein, wobei es sich bei Doppelbandelektroden als günstig erwiesen hat, wenn sie nicht parallel, sondern in verschiedenen Winkeln zueinander ausgeführt sind. Die Elektroden, speziell die Klebeelektroden, bei denen es sich üblicherweise um Wegwerfelektroden handelt, können auch dazu verwendet werden, um zB mittels RFID oder anderer Hilfsmittel identifiziert zu werden. Damit ist es im Sinne der Genauigkeit der Messergebnisse möglich, nur Elektroden zur Verwendung zuzulassen, mit denen das Gerät ursprünglich geeicht wurde und bei denen daher die notwendige entsprechende Qualität und Konfiguration gewährleistet ist.

Zusammengefasst ermöglicht die erfindungsgemäße Vorrichtung 2 eine Impedanzmessung von Körpersegmenten bei mehreren Frequenzen, falls zweckmäßig auch unter Verwendung eines Cole-Cole Plots, zur Bestimmung von intrazellulärem Wasser (bzw. Muskelmasse und Fettmasse) sowie von extrazellulärem Wasser, weiters die Impedanzmessung unter Aufteilung des Körpers in seine verschiedenen Segmente, z.B. Arme und Beine (oder auch nur Segmente der Arme und Beine), in den Rumpf und seine Teile, nämlich Thorax und Abdomen (da diese eine sehr unterschiedliche Zusammensetzung und damit unterschiedliche spezifische Widerstände aufweisen). Dies ist unter Verwendung von möglichst wenigen Speiseleitungen 5 zum Körper und möglichst wenigen Elektroden 8, 14 am Körper des Lebewesens 6 möglich. Die vorliegende Vorrichtung 2 kann die sogenannte 4-Punkt-Methode mit außen oder in Nachbarschaft liegenden Stromelektroden 8 und innen oder in Nachbarschaft liegenden Messelektroden 14 anwenden.

Die vereinten Elektrodenregionen 1 sind zur Anbringung an unterschiedlichen Körperteilen vorgesehen, so dass zwischen peripheren Elektrodenregionen (an den Enden der Extremitäten von lebenden Körpern) und zentralen Elektrodenregionen (am Rumpf, Hals, Kopfbereich liegend) unterschieden werden kann.

Periphere vereinte Elektrodenregionen werden aufgebracht an:
a) Fingern, Händen, Unterarmen zur Stromeinspeisung und Spannungsmessung (z.B. Einspeisung Finger, Hand, bzw. Hand und Unterarm zur Spannungsmessung an der linken und rechten oberen Extremität.
b) Zehen, Füßen, Unterschenkeln zur Stromeinspeisung bzw. Füßen und Unterschenkeln zur Spannungsmessung an der linken und rechten unteren Extremität.

Zentrale vereinte Elektrodenregionen werden aufgebracht an:
c) Kopf, Hals, oberem Brustkorb zur Stromeinspeisung bzw. zur Spannungsmessung. In diesem Bereich genügt eventuell auch nur eine Elektrode zur Spannungsmessung.
d) Unterem Brustkorb zur Stromeinspeisung und Spannungsmessung, wobei in diesem Bereich eventuell auch nur Elektroden für die Spannungsmessung ausreichen.

Andere Regionen des Körpers, die einem ähnlichen Messansatz unterzogen werden können, z.B. auch lokalisierte Körperregionen wie Teile des Schädels, des Halses, des Brustkorbs, des Abdomens, oder die Mamma, können ebenfalls zur Anbringung von vereinten Elektrodenregionen ausgewählt werden.

Aus den obigen Schilderungen ist klar ersichtlich, wie komplex und vielfältig die Vorrichtung 2 aufgebaut ist und funktioniert. Daher hat sich die Ausbildung der Stromumschalter 3 und Spannungsumschalter 10 als Multiplexer bewährt. Auch die Verwendung eines FPGA zum Schaltungsaufbau bringt eine große Vereinfachung mit sich. Um ein Vielkanal-EKG in die Messungen zu integrieren, muss die CPU 23 eine große Rechenkapazität besitzen und daher als schneller Prozessor ausgeführt sein, wobei auch die Datenspeicher ausreichend groß und schnell sein müssen. Zur Visualisierung der Messergebnisse sollte ein großer Bildschirm zur Anzeige der zahlreichen synchronisierten Daten und Kurven zur Verfügung stehen. Es können auch Schnittstellen zu bestehenden Arztsystemen vorgesehen sein. Die Daten sollten für die wissenschaftliche Analyse in geeigneten Datenformaten, z.B. Excel, oder anderen Datenbankformaten zur Verfügung gestellt werden. Sehr bewährt hat sich ein Datenspeicher, mit dessen Hilfe über den Zeitverlauf numerisch und/oder grafisch eine Änderung der Körperzusammensetzung, der Körperfunktionen und auch der Veränderungen des EKGs für den einzelnen Patienten registriert und grafisch ausgegeben wird. So kann dann mit empirischen Formeln, die an Hand von Goldstandardverfahren (wie z.B. Echocardiographie, biochemischer Parameter wie z.B. NTpro-BNP oder seiner Derivate bzw anderer biochemischer Parameter, die sich bei Herzschwäche verändern, Ergometrie, Spiroergometrie, Schwellen der Sauerstoffaufnahme, Lactatmessung, Ankle-Brachial Index, Arteriographie, Ganzkörper DXA, Deuterium- und Natriumbromid- Bestimmung) zur Erfassung von Körperfunktionen und Zusammensetzung, wie z.B. Herzleistung, Faserdehnung des Herzens , maximaler Leistungsfähigkeit, Schwellenwerte derselben, arterieller und venöser Durchblutung, von Muskelmasse, Fettmasse, Ganzkörperwasser, Extrazellulärraum und deren Abweichungen von der Norm kalibriert werden, indem obige Parameter z.B. mittels multiplen Regressionsgleichungen, welche eventuell anthropometrische Daten, sowie alle Impedanzdaten enthalten können, an Hand der Goldstandardverfahren geschätzt werden. Zur Erfassung von Über- oder Unterhydrierung oder auch von Sarkopenie bewährt sich in der Folge besonders die Berechnung der Abweichung von TBW oder ECW oder ICW oder ECW/ICW Ratio von der zwischen der FM/kg Körpergewicht einerseits und TBW/kg Körpergewicht bzw ECW/kg Körpergewicht bzw ECW/ICW bei Gesunden ermittelten Regressionsgeraden. Für die Bestimmung von Sarkopenie bewährt sich besonders die Ausgabe der "appendicular muscle mass", wie sie international Standard ist, das ist die Muskelmasse von Schulter und Armen einerseits, bzw von Hüfte und Beinen andererseits, und wie sie durch die segmentale Impedanzspektroskopie exzellent erfasst werden kann. Bewährt hat sich auch die Korrektur der so rechnerisch ermittelten Muskelmasse mittels des Korrektur für ein allenfalls gestörtes Verhältnis (zB Quotienten) zwischen Extrazellulärraum einerseits und Intrazellulärraum bzw. Ganzkörperwassers andererseits. So kann das Vorliegen einer Sarkopenie und das Grading derselben im Vergleich zu einem Normkollektiv ausgegeben werden.

Besonders interessant ist auch die Ausgabe und/oder graphische Darstellung des Zeitverlaufes der ermittelten Parameter, nicht nur nach einzelnen Interventionen wie z.B. Ergometrie, Schrittmachereinstellungen, pharmakologischen und physiologischen Interventionen, nicht nur bei einer einzelnen Untersuchung, sondern besonders auch bei wiederholten Untersuchungen in größeren Zeitabständen. Hier können zB automatisch nur Zeitverläufe selektioniert werden, bei denen die gemessenen oder errechneten Parameter sich signifikant und klinisch relevant seit der Letztaufzeichnung verändert haben. Klinisch relevant sind zB Veränderungen von Parametern, die aus dem bekannten Normbereich herauswandern, wie der PQ Zeit die Entstehung eines AV Blocks, der QT Dauer über oder unter den frequenzadaptierten Normbereich, von ST Senkung oder Hebung über den bekannten Normbereich, Änderungen des Herzvektors, der Amplutude und Richtung der T Welle, plötzliche Änderungen der Herzfrequenz, der sympathikovagalen Balance, errechnet der Power des 0,1 und des 0,3 Hz Bandes, bzw aus dem Verhältnis des 0,1 und 0,3 Hz Bandes der Herzratenvariabilität, plötzlich zwichenzeitlich aufgetretene Unterschiede der Segmente, der Volumswelle in den Beinen oder von Anstiegen der ECF/ICF oder ECF/TBW Ratios in einzelnen Körpersegmenten usw.. So werden unübersichtliche Verlaufsausdrucke verhindert.

## Patentansprüche

1. Vorrichtung (2) zur Impedanzmessung an Segmenten eines menschlichen oder tierischen Körpers, wobei die Impedanzmessung bevorzugt eine Multifrequenz-Impedanzmessung ist, wobei die Vorrichtung (2) eine Stromquelle (4), Stromumschalter (3), an Segmenten des menschlichen oder tierischen Körpers (6) anordenbare Stromelektroden (8), Spannungsumschalter (10) und Messelektroden (14) aufweist, wobei ein jeder Ausgang der Stromquelle (4) mit einem Eingang eines Stromumschalters (3) verbunden ist, wobei jeder Stromumschalter (3) eine Vielzahl von Ausgängen aufweist und der Eingang des jeweiligen Stromumschalters (3) umschaltbar mit einem seiner Ausgänge zusammenschaltbar ist, wobei die Ausgänge der Stromumschalter (3) mit elektrischen Speiseleitungen (5) der Stromelektroden (8) verbunden sind, wobei die Messelektroden (14) Spannungssignale erfassen und die Vorrichtung (2) aus dem von der Stromquelle (4) gelieferten Strom und den erfassten Spannungssignalen die Impedanzen der Segmente des menschlichen oder tierischen Körpers (6) ermittelt, wobei in die Speiseleitungen (5) nahe der oder unmittelbar an den Stromelektroden (8) Schaltvorrichtungen (7) eingebaut sind, mit denen die Stromelektroden (8) mit ihren Speiseleitungen (5) verbindbar und von ihnen trennbar sind, wobei die Vorrichtung (2) dazu konfiguriert ist, immer nur eine Messstrecke durch den Spannungsumschalter (10) freizugeben und durch Schalten der Stromumschalter (3) und der Schaltvorrichtungen (7) jeweils nur zwei Stromelektroden (8) mit der Stromquelle (4) zu verbinden und alle übrigen Stromelektroden (8) durch Schalten der Schaltvorrichtung (7) von der Speiseleitung (5) zu trennen und die Speiseleitung (5) durch Schalten der Stromumschalter (3) von der Stromquelle (4) zu trennen, wobei Signalleitungen (19) der Messelektroden (14) zu Eingängen von Spannungsumschaltern (10) geführt sind.

2. Vorrichtung nach Anspruch 1, **dadurch gekennzeichnet, dass** nahe oder unmittelbar an den Messelektroden (14) Spannungsverstärker (9) bzw. Spannungsfolger mit hohem Eingangswiderstand (9) in die Signalleitungen (19) eingefügt sind.

3. Vorrichtung nach Anspruch 1 oder 2, **dadurch gekennzeichnet, dass** die Schaltvorrichtungen (7) von der Vorrichtung (2) schaltbar sind.

4. Vorrichtung nach einem der Ansprüche 1 bis 3, **dadurch gekennzeichnet**, dassdie Spannungsumschalter (10) jeweils einen Ausgang aufweisen, der mit einem Eingang des Spannungsumschalters (10) zusammenschaltbar ist.

5. Vorrichtung nach Anspruch 4, **dadurch gekennzeichnet, dass** die Ausgänge der Spannungsumschalter (10) mit Eingängen eines Differenzverstärkers (11) verbunden sind, der aus den an seinen Eingängen anliegenden Spannungssignalen eine Differenzspannung (Udiff) ermittelt, die die Vorrichtung (2) für die Impedanzermittlung verwendet.

6. Vorrichtung nach einem der vorhergehenden Ansprüche, **dadurch gekennzeichnet, dass** zumindest eine der Stromelektroden (8) oder eine zusätzliche Elektrode (8a) über einen Widerstand (12) mit einem Ausgleichsstrom beaufschlagbar ist, wobei der Anschluss des Widerstands (12) an die Stromelektrode (8) zwischen der Schaltvorrichtung (7) und der Stromelektrode (8) angeordnet ist.

7. Vorrichtung nach einem der vorhergehenden Ansprüche, **dadurch gekennzeichnet, dass** eine Ausgleichsstromelektrode (8a) vorgesehen ist, die über einen Widerstand (12a) mit einem Ausgleichsstrom beaufschlagbar ist.

8. Vorrichtung nach einem der vorhergehenden Ansprüche, **dadurch gekennzeichnet, dass** jeweils eine Stromelektrode (8) und eine Messelektrode (14) auf einem gemeinsamen Elektrodenträger angeordnet zu einer vereinten Elektrodenregion (1) gruppiert sind, wobei die Stromeinspeisung immer mit zwei Stromelektroden (8), die je auf einer vereinten Elektrodenregion (1) gruppiert sind, erfolgt, wobei für die Spannungsmessung jedoch immer maximal nur eine Messelektrode (14), die auf einer der beiden vereinten Elektrodenregionen (1) liegt, verwendet wird.

9. Vorrichtung nach Anspruch 8, **dadurch gekennzeichnet, dass** die Vorrichtung (2) nur von maximal einer vereinten Elektrodenregion (1) die Stromelektrode (8) mit der Stromquelle (4) zusammenschaltet und die Messelektrode (14) zur Spannungsmessung verwendet.

10. Vorrichtung nach einem der Ansprüche 8 oder 9, **dadurch gekennzeichnet, dass** vereinte Elektrodenregionen (1) zur Anbringung an peripheren Körpersegmenten und an zentralen Körpersegmenten auswählbar sind, wobei vorzugsweise eine weitere vereinte Elektrodenregion (1) zum Anbringen an einer Körperregion auswählbar ist, an der die Brustwand-Elektroden V1 bis V6, bevorzugt V4 bis V6, eines EKGs platziert sind.

11. Vorrichtung nach einem der vorhergehenden Ansprüche, **dadurch gekennzeichnet, dass** an dem gemeinsamen Elektrodenträger ein Drucksensor zur Pulsmessung angeordnet ist, insbesondere eine flüssigkeitsgefüllte Blase, die mit einem Druckaufnehmer kommuniziert, wobei die flüssigkeitsgefüllte Blase vorzugsweise mittels einer gesteuerten hydraulischen oder elektromotorischen Anpressvorrichtung gegen ein Körperteil anpressbar ist.

12. Vorrichtung nach einem der vorhergehenden Ansprüche, **dadurch gekennzeichnet, dass** sie die Impedanz und eine Änderung der Impedanz mit dem Herzschlag in zumindest zwei Segmenten bestimmt, wobei die Segmente definiert sind durch
a) bezüglich der Einspeisung einerseits durch zwei an einem zentralen (Z) Körperteil und einem peripheren (P) Körperteil anlegbare vereinte Elektrodenregionen (1), und bezüglich der Spannungsmessung durch eine an dem am zentralen (Z) Körperteil anlegbare vereinte Elektrodenregion (1) und eine zentral liegende Einzelelektrode (20);
b) andererseits bezüglich der Stromeinspeisung durch zwei an peripheren (P) Körperteilen anlegbare vereinte Elektrodenregionen (1) und bezüglich der Spannungsmessung durch die periphere (P) vereinte Elektrodenregion (1) und eine zentrale Messelektrode, entweder als Einzelelektrode (20) oder als Messelektrode (14) einer zentralen (Z) vereinten Elektrodenregion vorliegend; wobei
c) Alternativ zu b) das zweite Körpersegment auch bezüglich der Stromeinspeisung definiert ist durch eine zentrale (Z) und eine periphere vereinte Elektrodenregion (1) und bezüglich der Spannungsmessung durch zwei periphere (P) vereinte Elektrodenregionen, von denen eine nicht vom Strom durchflossen ist.

13. Vorrichtung nach einem der vorhergehenden Ansprüche, **dadurch gekennzeichnet, dass** ein EKG-Gerät (21) in die Vorrichtung (2) integriert ist, wobei das EKG-Gerät (21) zumindest Extremitätenelektroden, vorzugsweise auch Brustwandelektroden, aufweist.

14. Vorrichtung nach Anspruch 13, **dadurch gekennzeichnet, dass** die Messelektroden (14) als EKG-Elektroden ausgebildet sind, indem Zweigleitungen (22) der Signalleitungen (19) der Messelektroden (14) zum EKG-Gerät (21) geführt sind.

15. Vorrichtung nach einem der vorhergehenden Ansprüche, **dadurch gekennzeichnet, dass** die Stromelektroden (8) und/oder die Messelektroden (14) als Messgrößenaufnehmer/Geber (17, 17a) für Beschleunigungswerte ausgebildet sind.

16. Vorrichtung nach einem der vorhergehenden Ansprüche, **dadurch gekennzeichnet, dass** sie eine CPU (23) aufweist, die mittels multipler Regressionsgleichungen bzw. mathematischer Modelle Parameter für Körperfunktionen und Zusammensetzung, beispielsweise die Herzleistung, biochemische Parameter derselben, für die körperliche Maximalleistung, für die aeroben und anaeroben Schwellen, für die Durchblutung der Körperteile, für Arterien- und Venenfunktion, z.B. den Ankle-Brachial Index, arterielle und venöse Thrombosen, bzw. arterielle Embolien, die Körperkompartimente, Ganzkörperwasser, Extrazellulärraum, Muskelmasse, Fettmasse, Verhältnis von Extrazellulärraum zu Intrazellulärraum oder Ganzkörperwasser im Ganzkörper und den einzelnen Körperteilen und all deren Abweichungen von der Norm ermittelt.

## Claims

1. Device (2) for impedance measurements on segments of a human or animal body, the impedance measurements preferably being a multi-frequency impedance measurements, wherein the device (2) comprises a current source (4), current changeover switches (3), current electrodes (8) placeable on segments of the human or animal body (6), voltage changeover switches (10) and measuring electrodes (14), wherein each output of the current source (4) is connected to an input of a current changeover switch (3), wherein each current changeover switch (3) has a plurality of outputs and the input of the respective current changeover switch (3) is switchably interconnectable with one of its outputs, wherein the outputs of the current changeover switches (3) are connected to electric feed lines (5) of the current electrodes (8), wherein the measuring electrodes (14) detect voltage signals and the device (2) determines the impedances of the segments of the human or animal body (6) from the current supplied by the current source (4) and the detected voltage signals,
wherein switching devices (7) are incorporated into the feed lines (5) near or directly at the current electrodes (8), with which switching devices (7) the current electrodes (8) may be connected to or disconnected from their feed lines (5), wherein the device (2) is configured to always open up only one measuring section via the voltage changeover switch (10) and respectively connect only two current electrodes (8) to the current source (4) by switching the current changeover switches (3) and the switching devices (7), and to disconnect all other current electrodes (8) from the feed line (5) by switching the switching device (7), and to disconnect the feed line (5) from the current source (4) by switching the current changeover switches (3) wherein signal lines (19) of the measuring electrodes (14) are connected to inputs of voltage switches (10).

2. Device according to claim 1, **characterized in that** voltage amplifiers (9) or voltage followers with a high input resistance (9) are integrated into the signal lines (19) near or directly at the measuring electrodes (14).

3. Device according to claim 1 or 2, **characterized in that** the switching devices (7) are switchable by the device (2).

4. Device according to one of the claims 1 to 3, **characterized in that** the voltage changeover switches (10) each have an output that is interconnectable with an input of the voltage changeover switch (10).

5. Device according to claim 4, **characterized in that** the outputs of the voltage changeover switches (10) are connected to inputs of a differential amplifier (11), which, based on the voltage signals applied to its inputs, determines a differential voltage (Udiff), which the device (2) uses for the determination of impedance.

6. Device according to one of the preceding claims, **characterized in that** a compensating current may be applied to at least one of the current electrodes (8) or an additional electrode (8a) via a resistor (12), wherein the connection of the resistor (12) to the current electrode (8) is positioned between the switching device (7) and the current electrode (8).

7. Device according to one of the preceding claims, **characterized in that** a compensating current electrode (8a) is provided, to which a compensating current may be applied via a resistor (12a).

8. Device according to one of the preceding claims, **characterized in that** one current electrode (8) and one measuring electrode (14), respectively, are arranged on a common electrode carrier grouped into a combined electrode region (1), wherein current feeding always takes place with two current electrodes (8) that are respectively grouped in a combined electrode region (1), wherein, however, not more than one measuring electrode (14) is used at a time, which is positioned on one of the two combined electrode regions (1).

9. Device according to claim 8, **characterized in that** the device (2) interconnects the current electrode (8) of no more than one combined electrode region (1) with the current source (4) and uses the measuring electrode (14) for voltage measurements.

10. Device according to one of the claims 8 or 9, **characterized in that** combined electrode regions (1) are selectable for attachment to peripheral body segments and central body segments, wherein preferably a further combined electrode region (1) is selectable for attachment to a body region where the chest wall electrodes V1 to V6, preferably V4 to V6, of an ECG are placed.

11. Device according to any of the preceding claims, **characterized in that** a pressure sensor for pulse measurement is arranged on the common electrode carrier, in particular a liquid-filled balloon communicating with a pressure transducer, wherein the liquid-filled balloon is pressable against a body part, advantageously by means of a controlled, hydraulic- or electric motor-driven pressing device.

12. Device according to one of the preceding claims, **characterized in that** it determines the impedance and impedance changes with the heartbeat of at least two body segments, the segments being defined
a) on the one hand, with regard to feeding by two combined electrode regions (1) positionable on a central (Z) body part and a peripheral (P) body part, and with regard to voltage measurements by a combined electrode region (1) positionable on a central (Z) body part and a centrally positioned single electrode (20);
b) on the other hand, regarding current feeding by two electrode regions (1) positionable on peripheral (P) body parts, and with regard to voltage measurements by the peripheral (P) combined electrode region (1) and a central measuring electrode, either as single electrode (20) or as measuring electrode (14) of a central (Z) combined electrode region; wherein
c) alternatively to b), the second body segment is also defined with regard to current feeding by a central (Z) and a peripheral combined electrode region (1), and with regard to voltage measurements by two peripheral (P) combined electrode regions, one of which does not carry any current.

13. Device according to one of the preceding claims, **characterized in that** an ECG device (21) is integrated into the device (2), wherein the ECG device (21) has at least extremities electrodes, preferably also chest wall electrodes.

14. Device according to claim 13, **characterized in that** the measuring electrodes (14) are implemented as ECG electrodes by providing branch lines (22) of the signal lines (19) of the measuring electrodes (14) to the ECG device (21).

15. Device according to one of the preceding claims, **characterized in that** the current electrodes (8) and/or the measuring electrodes (14) are implemented as transducers/generators (17, 17a) for acceleration values.

16. Device according to one of the preceding claims, **characterized in that** it has a CPU (23) that, by means of multiple regression equations or mathematical models, determines parameters of body functions and compositions, such as cardiac performance, biochemical parameters thereof, for the physical maximum performance, for the aerobic and anaerobic thresholds, for the circulation of body parts, for arterial and venous functions, e.g. the ankle brachial index, arterial and venous thromboses or arterial embolisms, the body compartments, whole-body water, extracellular space, muscle mass, fat mass, ratio between extracellular space and intracellular space or total-body water in the whole body and in individual body parts and their deviations from the norm.

## Revendications

1. Dispositif (2) de mesure de l'impédance sur des segments d'un corps humain ou animal, la mesure d'impédance étant de préférence une mesure d'impédance multifréquence, le dispositif (2) comprenant une source de courant (4), des commutateurs de courant (3), des électrodes de courant (8) pouvant être disposées sur des segments du corps humain ou animal (6), des commutateurs de tension (10) et des électrodes de mesure (14), chaque sortie de la source de courant (4) étant reliée à une entrée d'un commutateur de courant (3), chaque commutateur de courant (3) ayant une pluralité de sorties et l'entrée du commutateur de courant (3) respectif pouvant être connectée à l'une de ses sorties d'une manière commutable, les sorties des commutateurs de courant (3) étant reliées aux lignes d'alimentation électriques (5) des électrodes de courant (8), les électrodes de mesure (14) détectant des signaux de tension, et le dispositif (2) déterminant les impédances des segments du corps humain ou animal (6) à partir du courant fourni par la source de courant (4) et des signaux de tension détectés,
dans lequel des dispositifs de commutation (7) sont installés dans les lignes d'alimentation (5) à proximité ou directement au niveau des électrodes de courant (8), permettant de connecter et de déconnecter les électrodes de courant (8) de leurs lignes d'alimentation (5), le dispositif (2) étant configuré pour ne libérer qu'une seule section de mesure à la fois par le commutateur de tension (10) et, en actionnant les commutateurs de courant (3) et les dispositifs de commutation (7), pour ne relier que deux électrodes de courant (8) à la source de courant (4) à la fois, et, en actionnant le dispositif de commutation (7), pour déconnecter toutes les autres électrodes de courant (8) de la ligne d'alimentation (5), et, en actionnant les commutateurs de courant (3), pour déconnecter la ligne d'alimentation (5) de la source de courant (4), les lignes de signal (19) des électrodes de mesure (14) étant acheminées vers des entrées des commutateurs de tension (10).

2. Dispositif selon la revendication 1, **caractérisé en ce que** des amplificateurs de tension (9) ou des suiveurs de tension à haute résistance d'entrée (9) sont insérés dans les lignes de signal (19) à proximité ou directement au niveau des électrodes de mesure (14).

3. Dispositif selon la revendication 1 ou 2, **caractérisé en ce que** les dispositifs de commutation (7) sont commutables par le dispositif (2).

4. Dispositif selon l'une quelconque des revendications 1 à 3, **caractérisé en ce que** chaque commutateur de tension (10) possède une sortie pouvant être connectée à une entrée du commutateur de tension (10).

5. Dispositif selon la revendication 4, **caractérisé en ce que** les sorties des commutateurs de tension (10) sont reliées aux entrées d'un amplificateur différentiel (11) qui détermine une tension différentielle (Udiff) que le dispositif (2) utilise pour la détermination d'impédance, à partir des signaux de tension appliqués à ses entrées.

6. Dispositif selon l'une quelconque des revendications précédentes, **caractérisé en ce qu'**au moins une des électrodes de courant (8) ou une électrode supplémentaire (8a) peut être alimentée par un courant de compensation via une résistance (12), la connexion de la résistance (12) à l'électrode de courant (8) étant disposée entre le dispositif de commutation (7) et l'électrode de courant (8).

7. Dispositif selon l'une quelconque des revendications précédentes, **caractérisé en ce qu'**une électrode de courant de compensation (8a) est prévue, qui peut être alimentée par un courant de compensation via une résistance (12a).

8. Dispositif selon l'une quelconque des revendications précédentes, **caractérisé en ce que**, chaque fois, une électrode de courant (8) et une électrode de mesure (14) sont disposées sur un support d'électrode commun et sont regroupées pour former une région d'électrode combinée (1), dans lequel l'alimentation en courant est toujours réalisée avec deux électrodes de courant (8), chacune étant regroupée sur une région d'électrode combinée (1), dans lequel pour la mesure de tension, cependant, au plus une seule électrode de mesure (14), qui se trouve sur l'une des deux régions d'électrode combinées (1), est utilisée à la fois.

9. Dispositif selon la revendication 8, **caractérisé en ce que** le dispositif (2) connecte l'électrode de courant (8) à la source de courant (4) à partir d'au plus une seule région d'électrode combinée (1) et utilise l'électrode de mesure (14) pour la mesure de tension.

10. Dispositif selon l'une quelconque des revendications 8 ou 9, **caractérisé en ce que** des régions d'électrode combinées (1) sont sélectionnables pour la fixation sur des segments corporels périphériques et sur des segments corporels centraux, de préférence une région d'électrode combinée (1) supplémentaire étant sélectionnable pour la fixation sur une région du corps où sont placées les électrodes thoraciques V1 à V6, de préférence V4 à V6, d'un électrocardiogramme.

11. Dispositif selon l'une quelconque des revendications précédentes, **caractérisé en ce qu'**un capteur de pression pour la mesure du pouls est disposé sur le support d'électrode commun, notamment une poche remplie de liquide communiquant avec un transducteur de pression, la poche remplie de liquide de préférence pouvant être pressée contre une partie du corps au moyen d'un dispositif de pression contrôlé hydrauliquement ou par un moteur électrique.

12. Dispositif selon l'une des revendications précédentes, **caractérisé en ce qu'**il détermine l'impédance et une variation de l'impédance avec le rythme cardiaque dans au moins deux segments, les segments étant définis:
a) par rapport à l'alimentation, d'une part, par deux régions d'électrode combinées (1) pouvant être appliquées à une partie centrale (Z) du corps et à une partie périphérique (P) du corps, et par rapport à la mesure de tension, par une région d'électrode combinée (1) pouvant être appliquée à la partie centrale (Z) du corps et une électrode unique (20) située au centre;
b) d'autre part, par rapport à l'alimentation en courant, par deux régions d'électrode combinées (1) pouvant être appliquées à des parties périphériques (P) du corps, et par rapport à la mesure de tension, par la région d'électrode combinée (1) périphérique (P) et une électrode de mesure centrale, étant disponible soit en tant qu'électrode unique (20), soit en tant qu'électrode de mesure (14) d'une région d'électrode combinée centrale (Z); dans lequel
c) en alternative à b), le deuxième segment corporel est également défini par rapport à l'alimentation en courant par une région d'électrode combinée (1) centrale (Z) et une région d'électrode combinée périphérique, et par rapport à la mesure de tension, par deux régions d'électrode combinées périphériques (P), dont l'une n'est pas desservie par le courant.

13. Dispositif selon l'une quelconque des revendications précédentes, **caractérisé en ce qu'**un dispositif ECG (21) est intégré au dispositif (2), le dispositif ECG (21) comportant au moins des électrodes pour les membres et, de préférence, également des électrodes thoraciques.

14. Dispositif selon la revendication 13, **caractérisé en ce que** les électrodes de mesure (14) sont configurées comme des électrodes ECG, grâce aux lignes de dérivation (22) des lignes de signal (19) des électrodes de mesure (14) étant acheminées vers le dispositif ECG (21).

15. Dispositif selon l'une quelconque des revendications précédentes, **caractérisé en ce que** les électrodes de courant (8) et/ou les électrodes de mesure (14) sont configurées comme des transducteurs/transmetteurs de grandeur de mesure (17, 17a) pour les valeurs d'accélération.

16. Dispositif selon l'une quelconque des revendications précédentes, **caractérisé en ce qu'**il comporte un processeur (23) qui détermine des paramètres pour des fonctions corporelles et pour la composition, par exemple le débit cardiaque, des paramètres biochimiques de celui-ci, pour la performance physique maximale, pour les seuils aérobiques et anaérobiques, pour la circulation sanguine dans les parties du corps, pour la fonction artérielle et la fonction veineuse, par exemple l'indice cheville-bras, les thromboses artérielles et veineuses ou les embolies artérielles, les compartiments corporels, l'eau au corps entier, l'espace extracellulaire, la masse musculaire, la masse grasse, le rapport de l'espace extracellulaire à l'espace intracellulaire ou l'eau au corps entier dans tout le corps et dans les différentes parties du corps et toutes leurs déviations de la norme, au moyen d'équations de régression multiple ou de modèles mathématiques.
